# EUROPEAN PATENT SPECIFICATION

(11) **EP 2 260 901 B1**
(45) Date of publication and mention of the grant of the patent: **18.09.2013**
(21) Application number: 09728009.3
(22) Date of filing: 31.03.2009
(51) Int. Cl.: A61N 5/06

(54) **DEVICE FOR HAIR-GROWING**
VORRICHTUNG FÜR HAARWACHSTUM
DISPOSITIF DE CROISSANCE CAPILLAIRE

(30) Priority: 31.03.2008 JP 2008093584
(43) Date of publication of application: 15.12.2010
(60) Divisional application: 12153553.8
(73) Proprietor: Panasonic Corporation, Osaka 571-8501 (JP)
(72) Inventor: NAKAGAWA, Takehiro, 2-1-61 Shiromi, Chuo-ku, Osaka 540-6207 (JP); HAMADA, Chosei, 2-1-61 Shiromi, Chuo-ku, Osaka 540-6207 (JP)
(74) Representative: Appelt, Christian W.
(86) International application number: PCT/JP2009/056668
(87) International publication number: WO 2009/123196

(56) References cited:
- EP-A1- 2 133 120
- WO-A1-2007/097542
- WO-A1-2007/119946
- JP-A- 2007 090 051
- JP-A- 2008 029 811
- JP-A- 2008 246 144
- JP-T- 2006 501 960
- US-A1- 2004 147 984
- US-A1- 2006 161 226
- US-A1- 2006 293 728
- US-A1- 2007 173 912

## Description

### Technical Field

The present invention is directed to hair-growth devices, and more particularly to a hair-growth device utilizing light for hair-growth.

### Background Art

In the past, there has been a hair-growth method using a medical agent. Moreover, there has been a hair-growth method which grows hair by irradiating a skin with light having a wavelength of 890nm (see Journal of the Korean Society Plastic & Reconstructive Surgeons 2004 pages 1 to 8).

The above mentioned hair-growth method using a medical agent or light irritates mildly a skin or skin inner tissue, thereby promoting hair-growth. Therefore, a user performing the above mentioned hair-growth method is likely to feel uncomfortable.

In EP 2 133 120 A1 a hair growth control method and an apparatus is disclosed, wherein modulating light with wavelength between 600-1000 nm excluding a range of 870-910 nm is disclosed. The light in this wavelength range is used to enhance hair growth free from causing inflammation, yet safely and gentle to skin.

US 2006/0161226 A1 discloses an apparatus and a method for reducing follicular cell apoptosis, wherein light emitting diodes are used illuminating light to a surface area in the wavelength region between 600 and 1000 nm to decrease follicular apoptosis. In particular, an array of light emitting diode is used to achieve the desired effect by illuminating light in a plurality of wavelengths.

US 2006/0293728 A1 discloses a device for low intensity optical hair growth control, wherein radiation of a suitable spectrum is applied to the skin in one or more pulses of between 1 and 100 ms and with maximum fluencies on the skin between 1...12 J/cm2. The device comprises a source of electromagnetic radiation that emits in a wavelength range between 550 and 1200 nm and more particularly in the wave length range between 600 and 950 nm.

US 2007/0173912 A1 discloses a phototherapy with specific infrared light for treating skin disorders, wherein the method applies radiation of a particular energy to an exposed surface. In a preferred case, the application of energy is provided by laser light in a wave length region between 1.4 microns and 2.5 micron in order to treat, e.g., psoriasis.

WO 2007/119946 A1 discloses light emitting diodes with a wave length of 420-950 nm to activate cells for combinational treatments, wherein four laser diodes emit light in four regions of different wavelengths such that light is irradiated in mixtures and/or pulses.

### Disclosure of invention

The above-mentioned problems are solved by a hair growth device according to claim 1. Claims 2 and 3 refer to specifically advantageous realizations of the device of claim 1.

In view of the above insufficiency, the present invention has been aimed to propose a hair-growth device capable of growing a user's hair in a comfortable manner.

The hair-growth device in accordance with the present invention includes an irradiator configured to irradiate one's skin with light having a wavelength equivalent to a specific absorption wavelength of water of 1790 nm.

According to the invention, it is possible to provide hair-growth effect without using a medical agent. Additionally, the light having a wavelength equivalent to the specific absorption wavelength of water does not irritate a skin or skin inner tissue. Therefore, it is possible to grow a user's hair in a comfortable manner without injuring a skin.

According to the preferred embodiment, it is possible to emit the light having a wavelength of 1790 nm, which is equivalent to the specific absorption wavelength of water and produces an excellent effect for improvement of the hair-growth effect.

In a preferred embodiment, the hair-growth device includes a time controller configured to allow the irradiator to emit continuously the light for at least 15 minutes.

According to this preferred embodiment, it is possible to improve the hair-growth effect.

According to the invention, it is possible to provide hair-growth effect without using a medical agent. Additionally, the light having a wavelength equivalent to the specific absorption wavelength of water does not irritate a skin or skin inner tissue. Therefore, it is possible to grow a user's hair in a comfortable manner without injuring a skin.

### Brief Description of Drawings

FIG. 1 is a schematic view illustrating a hair-growth device of one embodiment in accordance with the present invention,
FIG. 2 is a schematic view illustrating an irradiator of the above hair-growth device,
FIG. 3A is a graph showing a transition of the number of hairs,
FIG. 3B is a graph showing a transition of the number of hairs,
FIG. 4A is a graph showing a transition of a thickness of a hair,
FIG. 4B is a graph showing a transition of a thickness of a hair,
FIG. 5A is a graph showing a transition of a length of a hair,
FIG. 5B is a graph showing a transition of a length of a hair, and
FIG. 6 is a graph showing a relation between a hair-growth effect and an irradiation time of light of a wavelength equivalent to a specific absorption wavelength.

### Best Mode for Carrying Out the Invention

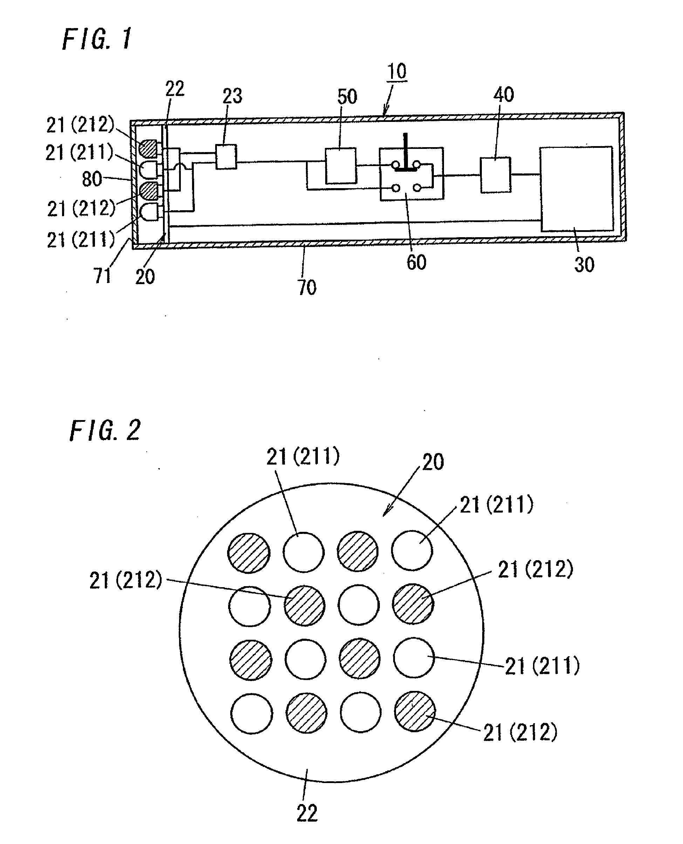

As shown in FIG. 1, the hair-growth device **10** of one embodiment of the present disclosure includes an irradiator **20,** a power source **30,** a timer **40,** a pulse generator **50,** a switching unit **60,** a housing **70,** and a cover **80.**

As shown in FIG. 2, the irradiator **20** includes a plurality (eight, in the illustrated instance) of first light sources **211** and a plurality (eight, in the illustrated instance) of second light sources **212.** Besides, when the first light source **211** does not need to be distinguished from the second light source **212,** the first light source **211** and the second light source **212** are referred to as a light source **21,** as necessary. In FIGS. 1 and 2, in order to distinguish the first light source **211** from the second light source **212,** the second light source **212** is illustrated with hatching.

The light source **21** is a light emitting diode. The first light source **211** is configured to emit light (hereinafter referred to as "first light", as necessary) having a wavelength of 950nm. The second light source **212** is configured to emit light (hereinafter referred to as "second light", as necessary) having a wavelength of 1450nm.

The first and second lights have wavelengths equivalent to specific absorption wavelengths of water. The specific absorption wavelength of water is defined as a wavelength which shows a strong absorption of light (remarkable change in light absorption) relative to other wavelengths when the light is absorbed in the water. In other words, the specific absorption wavelength of water is defined as a wavelength at a peak of an absorption spectrum of water. The specific absorption wavelength is thought to result from an O-H combination tone of water. Especially, the specific absorption wavelength in a near-infrared region is one of 950nm, 1150nm, 1450nm, and 1790nm, for example. Besides, the peak of the absorption spectrum of water has a width. Therefore, a wavelength of the light emitted from the irradiator **20** is not strictly equivalent to the specific absorption wavelength of water. In other words, a wavelength of the light emitted from the irradiator **20** may be in a range regarded as being equivalent to the specific absorption wavelength.

The light source **21** is mounted on a substrate **22.** As shown in FIG. 2, the substrate **22** is shaped into a circular disk shape. The eight first light sources **211** and the eight second light sources **212** are mounted on the substrate **22.** For example, the light sources **21** are mounted on in a four by four matrix manner. In this situation, the first light sources **211** and the second light sources **212** are arranged alternately.

The irradiator **20** includes a controller **23** configured to control the first light sources **211** and the second light sources **212.** For example, the controller **23** includes a first switch (not shown) interposed between the power source **30** and the first light sources **211,** a second switch (not shown) interposed between the power source **30** and the second light sources **212,** and a control circuit (not shown) configured to control the respective switches. Therefore, when the first switch is turned on, power is supplied from the power source **30** to the first light sources **211,** thereby turning on the first light sources **211.** When the second switch is turned on, power is supplied from the power source **30** to the second light sources **212,** thereby turning on the second light sources **212.** For example, the switches are semiconductor switching devices such as transistors, and the control circuit is a CPU or a logical circuit.

The controller **23** has first to fifth operation modes. In the first operation mode, the control circuit keeps turning off the both switches. In this situation, both the first light sources **211** and the second light sources **212** are kept turned off. In the second operation mode, the control circuit keeps turning on only the first switch. In this situation, only the first light sources **211** are kept turned on. In the third operation mode, the control circuit keeps turning on only the second switch. In this situation, only the second light sources **212** are kept turned on. In the fourth operation mode, the control circuit keeps turning on both the first switch and the second switch. In this situation, both the first light sources **211** and the second light sources **212** are kept turned on. In the fifth operation mode, the control circuit turns on the first switch and the second switch alternately. In this situation, the first light sources **211** and the second light sources **212** are turned on alternately.

As seen from the above, the controller **23** is configured to turn on the first light sources **211** and the second light sources **212** simultaneously and is configured to turn on the first light sources **211** and the second light sources **212** alternately.

The controller **23** includes a first operation unit (not shown) for manually switching among the five operation modes. The first operation unit is mounted on an outer surface of the housing **70.**

The power source **30** is configured to supply power for turning on the light source **21** to the irradiator **20**. For example, the power source **30** is a DC source configured to output a constant output voltage. A primary cell or a secondary cell can be adopted as the power source **30.** Alternately, the power source **30** may have a function of converting an AC voltage received from a commercial power source into a DC voltage.

The timer **40,** the switching unit **60,** and the pulse generator **50** are interposed between the power source **30** and the irradiator **20.**

The timer **40** is configured to break electrical connection between the power source **30** and the irradiator **20** when a predetermined time passes, for example. The timer **40** is defined as a time controller configured to allow the irradiator **20** to emit continuously light for at least 15 minutes.

The pulse generator **50** is configured to convert the output voltage of the power source **30** into a pulse voltage. In the present embodiment, the pulse voltage generated by the pulse generator **50** has a period of 500Hz. The period of the pulse voltage is not limited to 500Hz.

The switching unit **60** is configured to select an electric path for connection of the power source **30** and the controller **23** from a first electric path passing through the pulse generator **50** and a second electric path not passing through the pulse generator **50.** In the first power path, the pulse generator **50** is inserted into the electric path between the power source **30** and the controller **23.** In this situation, the pulse voltage is given to the irradiator **20.** Therefore, the light source **21** emits light intermittently (pulse lighting). In the second power path, the pulse generator **50** is not inserted into the electric path between the power source **30** and the controller **23.** In this situation, the constant output voltage is given to the irradiator **20.** Therefore, the light source **21** emits light continuously (continuation lighting).

In brief, the switching unit **60** is configured to switch between the pulse lighting and the continuation lighting. The switching unit **60** includes a second operation unit (not shown) for switching manually between the pulse lighting and the continuation lighting. The second operation unit is mounted on the outer surface of the housing **70.**

The housing **70** is configured to house the irradiator **20,** the power source **30,** the timer **40,** the pulse generator **50,** and the switching unit **60.** The housing **70** is shaped into a circular cylindrical shape. The housing **70** is provided in its first axial surface (left surface, in FIG. 1) with a window **71** for allowing light from the irradiator **20** to go outside. The irradiator **20** is housed in the housing **70** such that the respective light sources **21** are visible via the window **71.** The housing **70** has a diameter of 40mm, for example.

The cover **80** is made of a translucent material (e.g. a glass and a translucent resin) and is shaped into a circular disk shape. The cover **80** has enough dimensions to cover the window **71.** The cover **80** is fitted into the window **71.** The cover **80** is used for protection of the irradiator **20.** Besides, the cover **80** may be constructed to function as lens, as necessary.

A following first experimentation was performed in order to confirm hair-growth effect resulting from the aforementioned hair-growth device **10.**

In the first experimentation, three observed areas are provided to lower limbs of respective subjects A and B. Each of the observed areas is a square area of 4cm by 4cm. Hairs sprouted on the observed area were shaved prior to beginning the first experimentation. Two of the three observed areas were used as irradiation areas, and the other one was used as a control area (reference area). By use of the hair-growth device **10,** the first irradiation area was irradiated with light having a wavelength of 950nm, and the second irradiation area was irradiated with light having a wavelength of 1450nm. Additionally, in the first experimentation, the respective light sources **21** were turned on in the pulse lighting manner (at a frequency of 500Hz). The control area was not irradiated with light from the hair-growth device **10.**

Under the aforementioned condition, the irradiation area was irradiated with light continuously for 20 minutes every day over 5 consecutive days (20 minutes irradiation of light per day). A day next to a day when the irradiation is made first was defined as the first day. The observed areas were observed every 10 days from the first day.

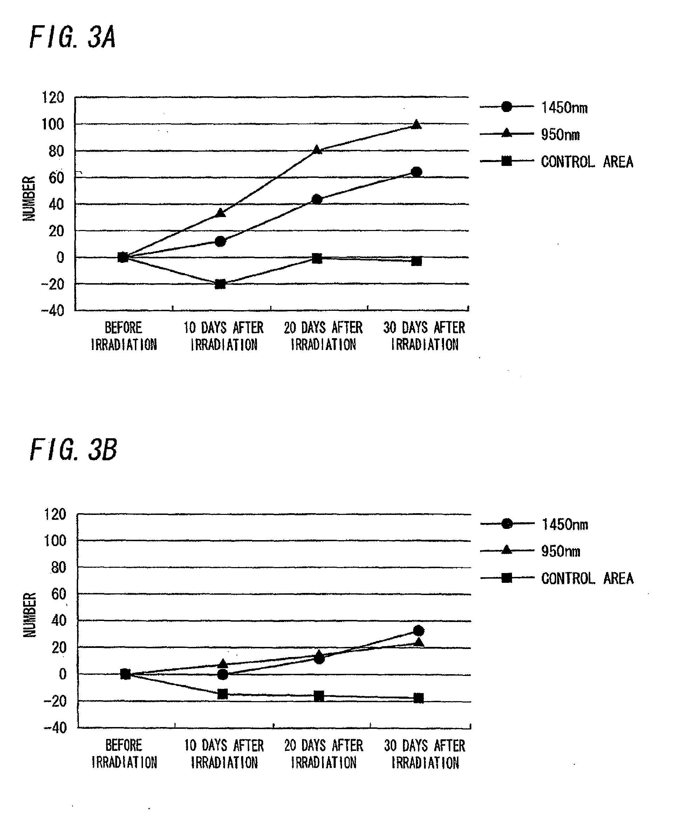

A result of the first experimentation is shown in FIGS. 3 to 5. Respective FIGS. 3A, 4A, and 5A show a result of the first experimentation with respect to the subject A, and respective FIGS 3B, 4B, and 5B show a result of the first experimentation with respect to the subject B.

Besides, an analysis of the result of the first experimentation was made by performing image processing of photos of the observed areas by use of a personal computer. In the above analysis, the number of hairs, a thickness of a hair, and a length of a hair in the observed area were evaluated. The thickness of a hair is defined as a thickness (diameter) of a base portion (i.e., a portion of a hair outside of the skin and nearest thereto).

FIGS. 3A and 3B are graphs showing a transition of the number of hairs with respect to elapsed days. A horizontal axis represents the elapsed days, and a vertical axis represents a variation of the number of hairs relative to a first initial value (the number of hairs just after being shaved).

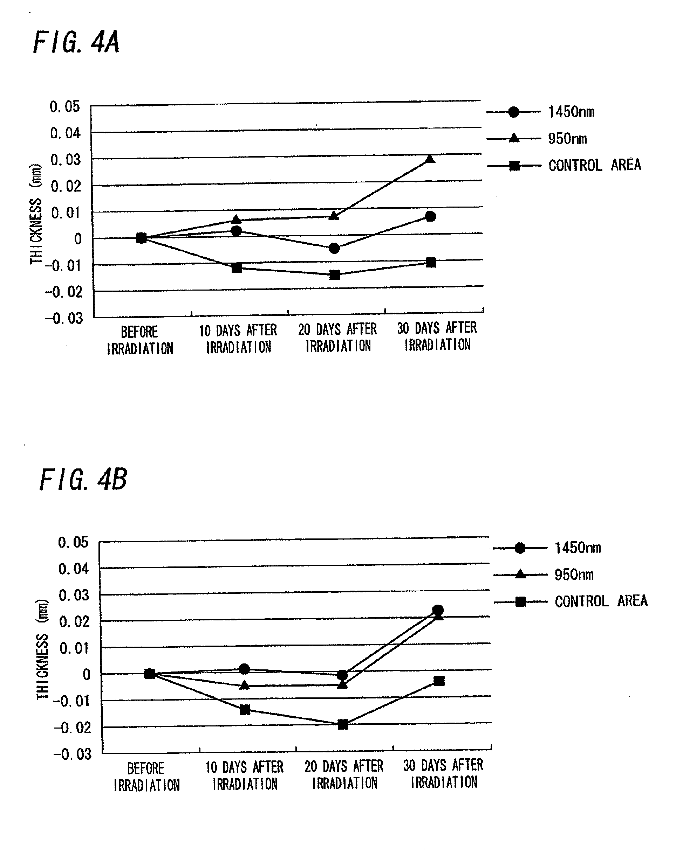

FIGS. 4A and 4B are graphs showing a transition of the thickness of a hair with respect to elapsed days. A horizontal axis represents the elapsed days, and a vertical axis represents a variation of an average of the thickness of a hair relative to a second initial value (average of the thickness of a hair just after being shaved).

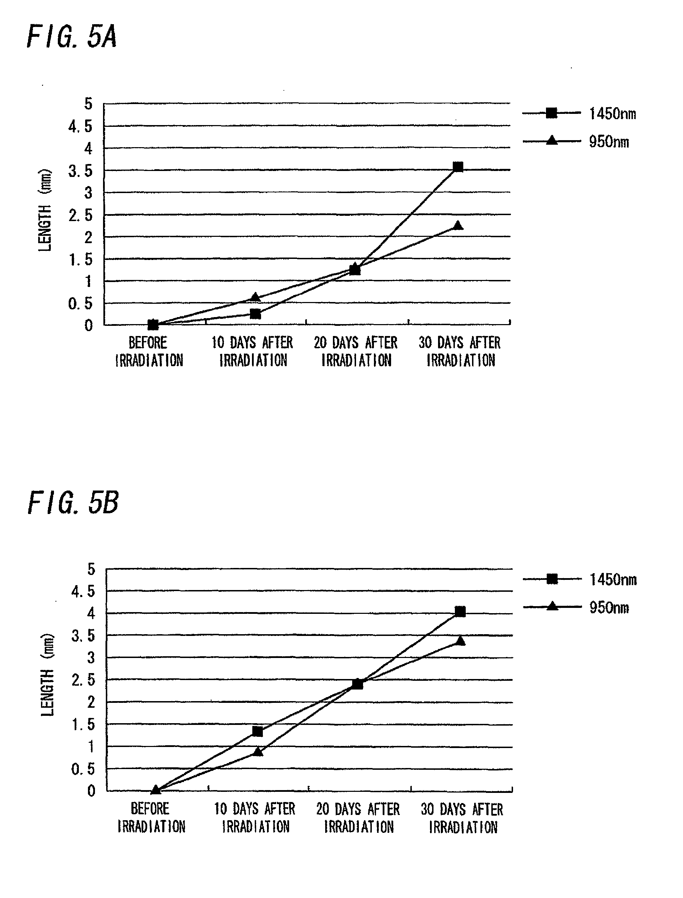

FIGS. 5A and 5B are graphs showing a transition of the length of a hair with respect to elapsed days. A horizontal axis represents the elapsed days, and a vertical axis represents a variation of the length of a hair relative to a third initial value (the length of a hair just after being shaved).

As shown in FIGS. 5A and 5B, results at 30 days after the irradiation with respect to both the subjects A and B show that the length of a hair of the second area was greater than that of the first area. That is, it indicated that, concerning the length of a hair, the second light produces superior hair-growth effect on both the subjects A and B in comparison with the first light.

As shown in FIGS. 3 and 4, results with respect to both the subjects A and B show that a difference between the control area and the irradiation areas appeared clearly.

FIG. 3A shows a result at 30 days after the irradiation with regard to the subject A that the number of hairs of the first irradiation area increases by as much as 100 times relative to the control area, and the number of hairs of the second irradiation area increases by as much as 60 times relative to that of the control area. FIG. 4A shows a result at 30 days after the irradiation that the thickness of a hair of the first irradiation area increases by as much as 0.04mm relative to the control area, and the thickness of a hair of the second irradiation area increases by as much as 0.02mm relative to that of the control area.

FIG. 3B shows a result at 30 days after the irradiation with regard to the subject B that the number of hairs of the first irradiation area increases by as much as 40 times relative to the control area, and the number of hairs of the second irradiation area increases by as much as 50 times relative to that of the control area. FIG. 4B shows a result at 30 days after the irradiation that the thickness of a hair of the first irradiation area increases by as much as 0.025mm relative to the control area, and the thickness of a hair of the second irradiation area increases by as much as 0.03mm relative to that of the control area.

The result of the first experimentation showed that growth of hair was promoted by irradiating skin with light having a wavelength equivalent to the specific absorption wavelength of water with relation to both the subjects A and B.

As apparent form the result of the first experimentation, a superior hair-growth effect can be obtained by irradiation a living body with light having a wavelength equivalent to the specific absorption wavelength of water. This could be explained by the following reason. The living body shows an efficient absorption reaction which results from an O-H combination tone of water when irradiated with the light of a wavelength equivalent to the specific absorption wavelength. In other words, the living body is activated by irradiation of light having the wavelength equivalent to the specific absorption wavelength. When the living body is so activated, the hair-growth is promoted.

Additionally, after the first experimentation, no skin disorders and no inflammation were not observed in the respective irradiation areas. This shows that light having a wavelength equivalent to the specific absorption wavelength of water does not cause abnormalities of a skin (flesh). Besides, the light source **21** was turned on in the pulse lighting manner in the first experimentation. When the light source **21** was turned on in the continuous lighting manner, a result similar to that of the first experimentation was obtained.

The result of the first experimentation shows that light having a wavelength of 950nm or 1450nm produces the distinguished hair-growth effect. Further, experimentation similar to the first experimentation was performed for light having a wavelength of 1150nm and light having a wavelength of 1790nm. This result shows that light having a wavelength of 1150nm or 1790nm produces the distinguished hair-growth effect in similar fashion as light having a wavelength of 950nm or 1450nm and did not cause inflammation of a skin. That is, light having a wavelength equivalent to the specific absorption wavelength of water produces hair-growth effect without injuring a skin.

The aforementioned hair-growth device **10** promotes the hair growth through a mechanism of that the light of the wavelength equivalent to the specific absorption wavelength of water is absorbed into a light absorption component already existing in the skin. The light absorption component absorbs more efficiently the light having a wavelength equivalent to the specific absorption wavelength rather than other light having a wavelength different from the specific absorption wavelength. Therefore, in contrast to the irradiation of light having a wavelength different from the specific absorption wavelength, the living body is activated to a greater extent by irradiation of the light having a wavelength equivalent to the specific absorption wavelength, and sees promotion of hair-growth.

As seen apparent from the above, the hair-growth device **10** can promote growth of hairs. Therefore, it is possible to provide hair-growth effect without using a medical agent. Additionally, in contrast to light having a wavelength of 890nm, the light having a wavelength equivalent to the specific absorption wavelength of water does not irritate a skin or skin inner tissue. Therefore, it is possible to grow a user's hair in a comfortable manner without injuring a skin.

It is possible to emit the light having a wavelength of 950nm or 1450nm, which is equivalent to the specific absorption wavelength of water and produces an excellent effect for improvement of the hair-growth effect. Besides, light emitted from the irradiator **20** may have a wavelength of 1150nm or 1790nm.

The result at 30 days after the irradiation with regard to the subject A shows that the first irradiation area was greater in the number of hairs and the thickness of a hair than the second irradiation area (see FIG. 3A and FIG. 4A).

By contrast, the result at 30 days after the irradiation with regard to the subject B shows that the second irradiation area was greater in the number of hairs and the thickness of a hair than the first irradiation area (see FIG. 3B and FIG. 4B).

As seen from the above, a comparison of the first irradiation area and the second irradiation area shows that the first light produces the superior hair-growth effect on the subject A than the second light, and that the second light produces the superior hair-growth effect on the subject B than the first light. Consequently, a wavelength of light optimal for hair-growth differs depending on a condition of a subject.

The subject A was a man in his twenties, and the subject B was a man in his fifties.

The result of the first experimentation shows that a wavelength of light optimal for hair-growth is different according to a user's age (that is, an age of a person to be irradiated with light affects the hair-growth effect).

In the fourth operation mode, the controller **23** turns on both the first light sources **211** and the second light sources **212** simultaneously. In the fifth operation mode, the controller **23** turns on the first light sources **211** and the second light sources **212** alternately.

Accordingly, the hair-growth device **10** can emit the first light of a wavelength of 950nm suitable for a man in his twenties and the second light of a wavelength of 1450nm suitable for a man in his fifties simultaneously or alternately.

Therefore, even if a wavelength of light optimal for hair-growth is varied depending on a condition (age) of a person to be irradiated with light, it is possible to promote efficiently and successfully hair-growth. That is, it is possible to provide the stable hair-growth effect irrespective of an age of a person to be irradiated with light.

Besides, the controller **23** has the second operation mode where only the first light sources **211** are kept turned on, and the third operation mode where only the second light sources **212** are kept turned on. Therefore, the light source **21** to be used can be selected in accordance with a user's age. Thus, it is possible to provide an optimal hair-growth effect according to a user's age.

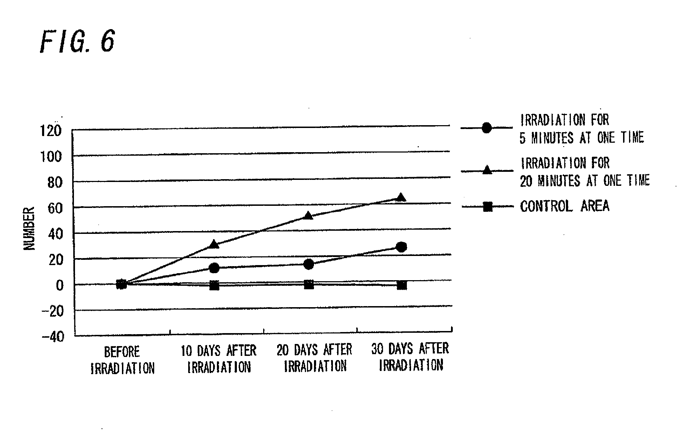

FIG. 6 shows a result of a second experimentation performed in order to confirm effect of the hair-growth device **10** of the present embodiment.

In the second experimentation, three observed areas are provided in a similar manner as the first experimentation. Two of the three observed areas were used as irradiation areas (third irradiation area and fourth irradiation area), and the other one was used as a control area (reference area). Both the third irradiation area and the fourth irradiation area were irradiated with light having a wavelength of 950nm by turning on the first light sources **211** in the pulse lighting manner at a frequency of 500Hz. Besides, the control area was not irradiated with light from the hair-growth device **10.**

Under the aforementioned condition, the third irradiation area was irradiated with light continuously for 20 minutes every day over 5 consecutive days (20 minutes irradiation of light per day). The fourth irradiation area was irradiated with light continuously for 5 minutes every day over 5 consecutive days (5 minutes irradiation of light per day). A day next to a day when the irradiation is made first was defined as the first day. The observed areas were observed every 10 days from the first day.

As shown in FIG. 6, both the third and fourth irradiation areas produced a higher hair-growth effect than the control area. Especially, the third area produced a higher hair-growth effect than the fourth irradiation area.

Further, skin disorders and inflammation were not observed in both the third and fourth irradiation areas. In the third and fourth irradiation areas, abnormalities of a skin did not occur. Besides, the first light sources **211** were turned on in the pulse lighting manner in the second experimentation. When the first light sources **211** were turned on in the continuous lighting manner, an effect similar to that of the second experimentation was obtained.

The result of the second experimentation shows that the hair-growth effect is enhanced as the continuous irradiation time of the light is increased. In the prior hair-growth method, the length of the continuous irradiation time of light is limited because light causes inflammation. In contrast, the hair-growth device **10** emits light having a wavelength equivalent to the specific absorption wavelength of water. Therefore, even if the continuous irradiation time of light increases, the skin does not suffer from inflammation or the like. That is, according to the hair-growth device **10,** it is possible to irradiate a skin with light for a long time without causing burn injury and inflammation in the skin.

Experimentations having the different continuous irradiation time were performed in a similar manner as the second experimentation. Results of these experimentations show that a superior hair-growth effect is observed when the continuous irradiation time is 15 minutes or more.

Therefore, the timer **40** is preferred to be configured to allow the irradiator **20** to emit light for at least 15 minutes. In this situation, a superior hair-growth effect can be obtained.

Besides, the light source **21** may be selected from an organic electroluminescent device, a discharge lamp, and a light bulb. In brief, the light source **21** may be configured to emit light having a wavelength equivalent to the specific absorption wavelength of water. For example, the first light sources **211** and the second light sources **212** may be arranged per groups each including the light sources having the same wavelength or arranged without being grouped by the wavelength. The first light sources **211** and the second light sources **212** need not be arranged at regular intervals. The number of the first light sources **211** need not be equivalent to the number of the second light sources **212.** The wavelength of the light from the irradiator **20** is not limited to the aforementioned instance, and may be selected from the specific absorption wavelength of water. The irradiator **20** may include the three or more types of the light sources **21** having wavelengths different from each other. The irradiator **20** may include only one type of the light sources **21.**

Besides, in the hair-growth device **10,** the timer **40,** the pulse generator **50,** and the switching unit **60** are optional. Additionally, in the controller **23,** the fourth operation mode and the fifth operation mode are optional.

Next, an explanation is made to a hair-growth method. The hair-growth method of the present embodiment includes a step of irradiating a skin with light having a wavelength equivalent to the specific absorption wavelength of water. According to the hair-growth method, it is possible to provide hair-growth effect without using a medical agent. Additionally, the light has a wavelength equivalent to the specific absorption wavelength of water. Therefore, the light does not irritate a skin or skin inner tissue. Thus, it is possible to grow a user's hair in a comfortable manner without injuring a skin.

In addition, the light emitted to the skin is preferred to be light having a wavelength of 950nm or 1450nm. In this situation, it is possible to emit the light having a wavelength of 950nm or 1450nm, which is equivalent to the specific absorption wavelength of water and produces an excellent effect for improvement of the hair-growth effect.

Preferably, in the step of irradiating the skin with the light, the first light having a wavelength of 950nm and the second light having a wavelength of 1450nm are emitted simultaneously or alternately. In this situation, the first light and the second light are used in combination to give the different hair-growth effects depending on an age of person to be irradiated with light. Therefore, it is possible to provide the stable hair-growth effect.

Preferably, the light is emitted to the skin continuously for at least 15 minutes. In this situation, it is possible to improve the hair-growth effect.

Preferably, the wavelength of the light emitted to the skin is selected on the basis of an age of a person to be irradiated with light. In this situation, it is possible to provide the hair-growth effect optimal for the age.

## Claims

1. A hair-growth device comprising an irradiator (20) configured to irradiate one's skin with light having a wavelength equivalent to a specific absorption wavelength of water,
**characterized in that**
the light has a wavelength of 1790 nm.

2. A hair-growth device as set forth in claim 1, wherein
said hair-growth device further includes a pulse generator (50),
said irradiator (20) including a light source (21) configured to emit the light, and
said pulse generator (50) being configured to give a pulse voltage to said irradiator (20) such that said light source (21) emits the light intermittently.

3. A hair-growth device as set forth in claim 1, wherein
said hair-growth device includes a time controller configured to allow said irradiator (20) to emit continuously the light for at least 15 minutes.

## Patentansprüche

1. Haarwuchsvorrichtung mit einer Strahlungsquelle (20), die ausgebildet ist, um auf die Haut eines Nutzers Licht mit einer Wellenlänge zu strahlen, die äquivalent ist zu einer spezifischen Absorptionswellenlänge von Wasser,
**dadurch gekennzeichnet, dass**
das Licht eine Wellenlänge 1790 nm aufweist.

2. Haarwuchsvorrichtung nach Anspruch 1, wobei
die Haarwuchsvorrichtung weiter einen Pulsgenerator (50) umfasst,
die Strahlungsquelle (20) eine Lichtquelle (21) umfasst, die ausgebildet ist, um Licht zu emittieren, und
der Pulsgenerator (50) ausgebildet ist, um eine Pulsspannung auf die Strahlungsquelle (20) anzuwenden, sodass die Lichtquelle (21) das Licht unterbrechend emittiert.

3. Haarwuchsvorrichtung nach Anspruch 1, wobei
die Haarwuchsvorrichtung einen Zeitgeber aufweist, der ausgebildet ist, um der Strahlungsquelle (20) zu erlauben, kontinuierlich Licht von zumindest 15 Minuten Länge zu emittieren.

## Revendications

1. Dispositif de croissance capillaire comprenant un irradiateur (20) configuré pour irradier la peau d'une personne avec de la lumière ayant une longueur d'onde équivalente à une longueur d'onde d'absorption spécifique de l'eau,
**caractérisé en ce que**
la lumière a une longueur d'onde de 1790 nm.

2. Dispositif de croissance capillaire selon la revendication 1, dans lequel
ledit dispositif de croissance capillaire comprend en outre un générateur d'impulsions (50),
ledit irradiateur (20) comprenant une source de lumière (21) configurée pour émettre la lumière, et
ledit générateur d'impulsions (50) étant configuré pour donner une tension d'impulsion audit irradiateur (20) de telle sorte que ladite source de lumière (21) émet de la lumière de façon intermittente.

3. Dispositif de croissance capillaire selon la revendication 1, dans lequel
ledit dispositif de croissance capillaire comprend un contrôleur de temps configuré pour permettre audit irradiateur (20) d'émettre la lumière en continu pendant au moins 15 minutes.
